## Europäisches Patentamt
## European Patent Office
## Office européen des brevets

(19)

(11) Numéro de publication: **0 080 412**
**B1**

(12)
# FASCICULE DE BREVET EUROPEEN

(45) Date de publication du fascicule du brevet:
**19.06.85**

(51) Int. Cl.⁴: **A 61 K 39/295**

(21) Numéro de dépôt: **82402104.2**

(22) Date de dépôt: **18.11.82**

(54) Vaccin anti-aphteux et anti-colibacillaire et son procédé de préparation.

(30) Priorité: **19.11.81 FR 8121676**

(43) Date de publication de la demande:
**01.06.83 Bulletin 83/22**

(45) Mention de la délivrance du brevet:
**19.06.85 Bulletin 85/25**

(84) Etats contractants désignés:
**CH DE GB IT LI**

(56) Documents cités:
**FR - A - 1 488 212**
**FR - A - 2 025 241**
**US - A - 4 140 763**
**US - A - 4 298 597**

**UNLISTED DRUGS, vol. 30, août 1978, page 123;**
**"Gletvax K88"**

(73) Titulaire: **RHONE MERIEUX, 17, rue Bourgelat,**
**F-69002 Lyon (FR)**

(72) Inventeur: **Desmettre, Philippe, 15 rue Benoît Tabard,**
**F-69130 Ecully (FR)**
Inventeur: **Favre, Hubert, Impasse des**
**Granges 17 avenue Franklin Roosevelt, F-69130 Ecully**
**(FR)**
Inventeur: **Riviere, Michel, Résidence des**
**Génovéfains 145 impase de Choulans, Porte D**
**F-69005 Lyon (FR)**

(74) Mandataire: **Lemoine, Michel et al, Cabinet Michel**
**Lemoine 13 Boulevard des Batignolles, F-75008 Paris**
**(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

La présente invention a trait à un vaccin anti-aphteux et anti-colibacillaire permettant l'immunisation d'animaux et notamment de bovins, contre la fièvre aphteuse et contre la colibacillose. L'invention a également trait à un procédé pour la préparation de ces vaccins.

On sait que la fièvre aphteuse des bovins peut être efficacement combattue par les vaccins anti-aphteux actuellement disponibles, obtenus à partir de virions aphteux. Il existe également des vaccins efficaces obtenus à partir de virions pour les porcins.

La procédure de vaccination comporte en général une vaccination annuelle régulière réglementée. Cette procédure permet d'obtenir, avec les vaccins précités, une immunisation efficace. Cependant, l'immunisation obtenue n'est pas immédiate, notamment après la première vaccination, et peut demander, avec les vaccins actuels, une durée de 15 à 20 jours environ après la vaccination. Ce délai, s'il n'est pas gênant d'une façon générale, peut cependant se révéler préjudiciable, par exemple lorsqu'une épizootie s'est déclarée.

On a proposé récemment de réaliser des vaccins purifiés contre la fièvre aphteuse à base d'une protéine virale provenent du virus aphteux et appelée V.P. thréonine, obtenue soit à partir de virions, soit par recombinaisons génétiques. La diffusion de tels vaccins se heurte cependant au pouvoir faiblement immunogène de cette protéine.

On connaît par ailleurs un antigène d'attachement présent sur certaines souches de Escherichia coli, appelé K 99 (K 88 pour les colibacilles porcins). Cet antigène est considéré comme responsable de l'attachement des bactéries sur la muqueuse intestinale et ses propriétés ainsi que ses procédés de préparation sont décrits par exemple dans la publication américaine Infection and Immunity, Jan. 1977, Vol. 15, N° 1, p. 272—279; Nov. 1978, Vol. 22, N° 2, p. 555—559; Sept. 1980, Vol. 29, N° 3, p. 1125—1133.

La présente invention a pour but de fournir des vaccins anti-aphteux et anti-colibacillaires qui permettent à la fois de remédier aux inconvénients précités et de fournir une immunisation efficace contre la fièvre aphteuse et contre la colibacillose néonatale des ruminants ou des porcins.

L'invention repose sur la constatation surprenante que des virions ou protéines virales aphteux peuvent être associés audit antigène d'attachement pour former un vaccin bivalent stable et efficace et que les vaccins ainsi obtenus, tout en conférant une immunité anti-colibacillaire, améliorent et augmentent l'immunogénicité de la valence aphteuse.

L'invention a donc pour objet un vaccin bivalent anti-aphteux et anti-colibacillaire, caractérisé par le fait qu'il comporte, dans un véhicule convenable, d'une part des antigènes vaccinants anti-aphteux obtenus à partir de virus, d'autre part des antigènes d'attachement K 99 (ou K 88 pour les vaccins pour les porcins).

De préférence, la proportion d'antigènes anti-aphteux par rapport aux antigènes d'attachement K 99 est de $10^7$ à $10^8$ DECP 50 en titre viral aphteux pour un titre d'antigène K 99 équivalent à 20 à 40 milliards de bactéries. Ces titres sont de préférence présentés par dose de 5 ml.

Les antigènes viraux vaccinants anti-aphteux sont de préférence des antigènes présents dans les vaccins classiques anti-aphteux. Ils comportent généralement des virions entiers. Ils peuvent comprendre l'une quelconque des valences aphteuses O, A, C ou toutes combinaisons de ces valences.

Le vaccin selon l'invention peut comporter un adjuvant classique de l'immunité tel qu'une émulsion ou un gel d'alumine ou de la saponine. De préférence, il comporte 1,7 mg d'alumine et 0,6 mg des saponine, par dose.

L'invention a également pour objet un vaccin bivalent anti-aphteux et anti-colibacillaire caractérisé par le fait qu'il comporte, dans un véhicule convenable, d'une part la protéine aphteuse V.P. thréonine, d'autre part l'antigène d'attachement colibacillaire K 99 (ou K 88 dans le cas d'un vaccin pour les porcins).

La proportion de protéine virale par rapport à l'antigène d'attachement est de préférence de 100 à 300 µg de protéine virale aphteuse pour 0,3 à 2 mg d'antigène K 99, par exemple 250 µg de protéine our 1 mg d'antigène K 99.

On doit comprendre que le véhicule contenant les antigènes est tel que les antigènes aphteux d'une part et les antigènes d'attachement d'autre part se trouvent associés dans une même phase aqueuse du véhicule. Les particules ou molécules peuvent ainsi réagir et d'une façon générale, on préfère que les particules ou protéines virales soient portées par l'antigène d'attachement constituant en quelque sorte une protéine porteuse de l'antigène aphteux.

L'invention a également pour objet un procédé de préparation d'un vaccin bivalent anti-aphteux et anti-colibacillaire, caractérisé par le fait que l'on mélange, dans une même phase, l'antigène viral anti-aphteux et l'antigène d'attachement K 99 (ou K 88).

L'invention a également pour objet un procédé de préparation d'un vaccin bivalent anti-aphteux et anti-colibacillaire, caractérisé par le fait que l'on mélange dans une même phase la protéine virale V.P. thréonine et l'antigène d'attachement K 99 (ou K 88).

De préférence, pour la préparation d'un vaccin contenant des virons à la façon de vaccins anti-aphteux classiques, on prépare le vaccin anti-aphteux classique sans procéder à une adsorption sur gel d'alumine ou autre, on ajoute la suspension convenable de facteur d'attachement K 99 puis l'on procède à l'adsorption.

Cependant, on peut également préparer un vaccin anti-aphteux habituel, le laisser décanter et remplacer le liquide surnageant (en général de l'ordre de 10%) par une suspension concentrée contenant les facteurs d'attachement K 99.

Pour préparer les antigènes d'attachement, on peut utiliser des bactéries inactivées portant les facteurs d'attachement, par exemple les souches décrites dans la litérature précitée, de sorte que le vaccin contiendra ces bactéries. En variante, on peut également, selon des techniques connues, préparer des suspensions contenant principalement les facteurs d'attachement K 99 ou K 88 à l'exclusion des bactéries.

Les vaccins selon l'invention peuvent être avantageusement présentés sous forme de doses individuelles de 5 ml.

D'autres avantages et caractéristiques de l'invention apparaîtront à la lecture de la description suivante, faite à titre d'exemples non limitatifs.

## Exemple 1

### Vaccin anti-aphteux O. A. C. et anti-colibacillaire

Le vaccin aphteux est produit à partir de virus multiplié soit sur explants d'épithélium lingual,soit sur culture cellulaire en suspension (voir DUBOUCLARD C. et coll. Vaccin aphteux sur épithélium. Bull. Soc. Sci. Vét. Lyon 1966, 68 [3], 243—253, ou FAURE H. et coll. Dév. Biol Standard 1976, 35, 409—423). L'inactivation est effectuée soit par le formol à 0,02% (V/V), soit par l'éthylèneimine à 0,05% (P/V). Le virus incativé est alors adsorbé sur gel d'alumine ($Al^{+++}$ 1,7 mg) et de la saponine est ajoutée à 0,6 mg/ml.

On prépare une suspension concentrée d'Escherichia coli entéro-toxique de souche O = 101 K 99 n° IFFA 15—192 (ACRES S. D., ISSACSON R. E., BABINK L. A., KAPITANY R. A., Immunization of calves against enterotoxigenic colibacillosis by vaccinating dams with purified K 99 antigen and whole cell bacterins Inf. Immunity, 1979, 25, 121—126) à une concentration $150 \times 10^9$ équivalents bactéries/ml et l'on inactive les bactéries, qui portent les facteurs d'attachement K 99, au formol à 4%oo.

Le vaccin selon l'invention est préparé en laissant décanter le vaccin aphteux obtenu, en évacuant le liquide surnageant d'un volume d'environ 10% et en le remplaçant par un même volume de la suspension concentrée de bactéries inactivées précitée.

On a effectué une vaccination de trois lots de cinq bovins. Le premier lot est soumis à la vaccination à l'aide du vaccin anti-aphteux tel que préparé ci-dessus.

Le deuxième lot est soumis à une vaccination par un vaccin colibacillaire constitué par la suspension concentrée de bactéries inactivées précitée et le troisième lot est soumis à la vaccination à l'aide du vaccin selon l'invention. Le tableau I donne le résultat des titrages, les anti-corps de la fièvre aphteuse (Ac O, A ou C) étant titrés en log 10 de séroneutralisation sur cellules et les anticorps K 99 étant titrés par agglutination bactérienne (log 10).

Tableau I

| Vaccin | Jour 7 | Jour 14 | Jour 21 |
|---|---|---|---|
| Lot 1 (Vaccin anti-aphteux) | | | |
| Ac O | 0,91 | 1,87 | 1,80 |
| A | 0,53 | 1,90 | 1,87 |
| C | 1,58 | 2,47 | 1,70 |
| K 99 | 0,6 | 0,6 | 0,8 |
| Lot 2 (Vaccin K 99) | | | |
| O | <0,24 | <0,24 | <0,24 |
| A | <0,24 | 0,24 | 0,24 |
| C | =0,67 | =0,60 | =0,60 |
| K 99 | 1,14 | 2,8 | 2,8 |

Suite de tableau I

| Vaccin | Jour 7 | Jour 14 | Jour 21 |
|--------|--------|---------|---------|
| Lot 3<br>(Vaccin selon l'invention) | | | |
| O | 1,58 | 1,90 | 1,61 |
| A | 0,98 | 2,06 | 1,88 |
| C | 1,78 | 2,48 | 1,66 |
| K 99 | 1,66 | 2,6 | 2,6 |

On voit que, pour un taux d'anticorps anti-colibacillaires à peu près similaire par rapport au vaccin anti-colibacillaire, le vaccin selon l'invention présente une augmentation rapide du taux d'anticorps anti-aphteux le septième jour.

Le tableau II ci-dessous représente une évaluation de la protection contre la fièvre aphteuse (en %) par corrélation titre anticorps/protection des bovins.

Tableau II

| | Jour 7 | Jour 14 | Jour 21 |
|--------|--------|---------|---------|
| Lot 1<br>(Vaccin anti-aphteux) | | | |
| O | 70% | 99% | 99% |
| A | 50% | 99% | 99% |
| C | 95% | 99% | 99% |
| Lot 2<br>(Vaccin selon l'invention) | | | |
| O | 98% | 99% | 98% |
| A | 78% | 99% | 99% |
| C | 99% | 99% | 98% |

Au septième jour, donc précocement, l'innocuité anti-aphteuse s'installe beaucoup plus fortement, les vaccins devenant ensuite comparables vers le quatorzième jour. Le vaccin selon l'invention permet donc une protection précoce.

## Exemple 2

### Vaccin à V.P. thréonine

On produit une V.P. thréonine par extraction du virus aphteux (selon BACHRACH H. L., MORGAN D. O., MOORE D. M., Foot and Mooth Disease virus immunogenic capsid protein VPT: N-terminal sequences and immunogenic peptides by CNBR and tryptic cleavages. Intervirology, 1979, 12 (2), 65—72), ou par recombinaison génétique (selon BROOKSBY J. B., Genetic engineering and Foot and Mooth Disease vaccine Nature, 1981, 289, p. 5798). La solution contenant la V.P. thréonique qui est obtenue est ajustée à la concentration requise pour obtenir 250 µg de V.P. thréonine pour 1 mg d'antigène K 99. On ajoute à la solution la quantité d'antigène K 99. Après avoir laissé la protéine et l'antigène interréagir, on effectue une dispersion dans un excipient huileux usuel faisant office d'adjuvant.

# 0 080 412

## Revendications

1. Vaccin bivalent anti-aphteux et anti-colibacillaire, caractérisé en ce qu'il comporte, dans un véhicule convenable, d'une part des antigènes vaccinants anti-aphteux obtenus à partir de virus, d'autre part des antigènes d'attachement K 99 d'Escherichia coli.

2. Vaccin selon la revendication 1, caractérisé en ce que la proportion d'antigènes anti-aphteux par rapport aux antigènes d'attachement K 99 est de $10^7$ à $10^8$ DECP 50 pour un équivalent de 20 à 40 milliards de bactéries.

3. Vaccin selon l'une quelconque des revendications 1 et 2, caractérisé en ce que les antigènes anti-aphteux comprennent au moins l'une des valences aphteuses O, A, C.

4. Vaccin selon l'une quelconque des revendications 1 à 3, caractérisé en ce qu'il comporte un gel d'alumine et de la saponine.

5. Vaccin selon l'une quelconque des revendications 1 à 4, caractérisé en ce que le antigènes anti-aphteux et d'attachement sont contenus dans une même phase.

6. Vaccin anti-aphteux et anti-colibacillaire, caractérisé en ce qu'il comporte, dans un véhicule convenable et dans une même phase, d'une part la protéine virale aphteuse V.P. thréonine, d'autre part les antigènes d'attachement K 99 d'Escherichia coli.

7. Vaccin selon la revendication 6, caractérisé en ce que la proportion de protéines virales par rapport aux antigènes d'attachement est de 100 à 300 µg de protéine pour 0,3 à 2 mg d'antigène K 99.

8. Vaccin selon l'une quelconque des revendications 6 et 7, caractérisé en ce que la phase contenant la protéine virale et l'antigène K 99, est dispersée dans un adjuvant huileux.

9. Vaccin anti-aphteux et anti-colibacillaire pour les porcins selon l'une quelconque des revendications 1 à 8, modifiée en ce que l'antigène d'attachement K 99 est remplacé par l'antigène d'attachement K 88.

10. Procédé de préparation d'un vaccin bivalent anti-aphteux et anti-colibacillaire selon l'une quelconque des revendications 1 à 5, caractérisé en ce que l'on mélange, dans une même phase, l'antigène viral anti-aphteux et l'antigène d'attachement K 99.

11. Procédé de préparation d'un vaccin bivalent anti-aphteux et anti-colibacillaire selon l'une quelconque des revendications 6 à 8, caractérisé en ce que l'on mélange dans une phase la protéine virale V.P. thréonine et l'antigène d'attachement K 99.

12. Procédé selon l'une quelconque des revendications 10 et 11 pour la préparation d'un vaccin bivalent anti-aphteux et anti-colibacillaire pour les porcins selon la revendication 9, modifiée en ce que l'on mélange, dans une même phase, l'antigène anti-aphteux ou la protéine virale V.P. thréonine et l'antigène d'attachement K 88.

13. Procédé selon l'une quelconque des revendications 10 à 12, caractérisé en ce que l'on effectue une adsorption du mélange.

14. Procédé selon l'une quelconque des revendication 10 à 13, caractérisé en ce que l'on utilise des bactéries entières inactivées d'Escherichia coli portant l'antigène d'attachement.

15. Procédé selon l'une quelconque des revendications 10 à 13, caractérisé en ce que l'on utilise pour l'antigène d'attachement une suspension l'antigène d'attachement sensiblement exempte de bactéries.

## Patentansprüche

1. Bivalenter anti-aphthöser und anti-colibazillärer Impfstoff, dadurch gekennzeichnet, daß er in einem geeigneten Vehikel einesteils anti-aphthöse Impfantigene, die aus Viren erhalten wurden, und andererseits Anlagerungsantigene K 99 von Escherichi coli enthält.

2. Impfstoff nach Anspruch 1, dadurch gekennzeichnet, daß das Verhältnis von anti-aphthösen Antigenen zu den Anlagerungsantigenen K 99 $10^7$ bis $10^8$ DECP 50 auf ein Äquivalent von 20 bis 40 Milliarden Bakterien beträgt.

3. Impfstoff nach einem der Ansprüche 1 oder 2, dadurch gekennzeichnet, daß die anti-aphthösen Antigene wenigstens eine der aphthösen Valenzen O, A, C umfassen.

4. Impfstoff nach einem der Ansprüche 1 bis 3, dadurch gekennzeichnet, daß er ein Alumina-Gel und Saponin enthält.

5. Impfstoff nach einem der Ansprüche 1 bis 4, dadurch gekennzeichnet, daß die anti-aphthösen und die Anlagerungsantigene in der gleichen Phase enthalten sind.

6. Anti-aphthöser und anti-colibazillärer Impfstoff, dadurch gekennzeichnet, daß er in einem geeigneten Vehikel und in der gleichen Phase einerseits das aphthöse virale Protein V.P. Threonin und andererseits die Anlagerungsantigene K 99 von Escherichia coli enthält.

7. Impfstoff nach Anspruch 6, dadurch gekennzeichnet, daß das Mengenverhältnis von Virusproteinen zu den Anlagerungsantigenen zwischen 100 und 300 µg Protein auf 0,3 bis 2 mg Antigen K 99 beträgt.

8. Impfstoff nach einem der Ansprüche 6 oder 7, dadurch gekennzeichnet, daß die das Virusprotein und das Antigen K 99 enthaltende Phase in einem öligen Hilfsstoff dispergiert ist.

5

9. Anti-aphthöser und anti-colibazillärer Impfstoff für Schweine nach einem der Ansprüche 1 bis 8, wobei das Anlagerungsantigen K 99 durch das Anlagerungsantigen K 88 ersetzt ist.

10. Verfahren zur Herstellung eines bivalenten anti-aphthösen und anti-colibazillären Impfstoff nach einem der Ansprüche 1 bis 5, dadurch gekennzeichnet, daß man das virale anti-aphthöse Antigen und das Anlagerungsantigen K 99 in der gleichen Phase mischt.

11. Verfahren zur Herstellung eines bivalenten anti-aphthösen und anti-colibazillären Impfstoffs nach einem der Ansprüche 6 bis 8, dadurch gekennzeichnet, daß man das Virusprotein V.P. Threonin und das Anlagerungsantigen K 99 in einer Phase mischt.

12. Verfahren zur Herstellung eines bivalenten anti-aphthösen und anti-colibazillären Impfstoffs für Schweine nach Anspruch 9 gemäß einer Modifikation der Ansprüche 10 oder 11, wobei man das anti-aphthöse Antigen oder das Virusprotein V.P. Threonin und das Anlagerungsantigen K 88 in der gleichen Phase mischt.

13. Impfstoff nach einem der Ansprüche 10 bis 12, dadurch gekennzeichnet, daß man eine Adsorption des Gemischs durchführt.

14. Impfstoff nach einem der Ansprüche 10 bis 13, dadurch gekennzeichnet, daß man ganze inaktivierte Escherichia coli-Bakterien, die das Anlagerungsantigen tragen, verwendet.

15. Impfstoff nach einem der Ansprüche 10 bis 13, dadurch gekennzeichnet, daß man für das Anlagerungsantigen eine Anlagerungsantigen-Suspension verwendet, die praktisch bakterienfrei ist.

**Claims**

1. Bifunctional anti-foot-and-mouth and anti-colibacillosis vaccine, characterised in that it comprises, in an acceptable vehicle on the one hand anti-foot-and-mouth vaccinating antigens obtained starting from virus, on the other hand antigens of Escherichia coli type K 99.

2. A vaccine according to claim 1, characterised in that the proportion of anti-foot-and-mouth antigens in relation to antigens of type K 99 is from $10^7$ to $10^8$ DECP 50 for an equivalent of 20 to 40 milliards of bacteria.

3. Vaccine according to any one of claims 1 and 2, characterised in that the anti-foot-and-mouth antigens comprise at least one of the foot and moth valences O, A, C.

4. Vaccine according to any one of claims 1 to 3, characterised in that it comprises a gel of alumine and of saponine.

5. Vaccine according to any one of claims 1 to 4, characterised in that the anti-foot-and-mouth and other antigens are contained in a single phase.

6. Anti-foot-and-mouth and anti-colibacillosis vaccine, characterised in that it comprises an acceptable vehicle and in a single phase, on the other hand type K 99 Escherichia coli antigens.

7. Vaccine according to claim 6, characterised in that the proportion of viral protein in relation to the other antigens is from 100 to 300 µg of protein 0.3 to 2 mg of antigen K 99.

8. Vaccine according to any one of claims 6 and 7, characterised in that the phase containing the viral protein and antigen K 99, is dispersed in an oily adjuvant.

9. Anti-foot-and-mouth and anti-colibacillosis vaccine for swine according to any one of claims 1 to 8, modified in that the type K 99 antigen is replaced by type K 88 antigen.

10. A process of preparation of an anti-foot-and-mouth and anti-colibacillosis vaccine according to any one of claims 1 to 5, characterised in that one mixes in a single phase, the anti-foot-and-mouth viral antigen and the type K 99 antigen.

11. A process of preparation of a bifunctional anti-foot-and-mouth and anti-colibacillosis vaccine according to any one of claims 6 to 8, characterised in that one mixes in one phase the viral protein VP threonine and the antigen type K 99.

12. A process of according to any one of claims 10 and 11 for the preparation of a bifunctional anti-foot-and-mouth and anti-colibacillosis vaccine for swine according to claim 9, modified in that one mixes in a single phase, the anti-foot-and-mouth antigen or the viral protein VP threonine and the antigen type K 88.

13. A process according to any one of claims 10 to 12, characterised in that one carries out an absorption of the mix.

14. A process according to any one of claims 10 to 13, characterised in that one uses whole inactivated bacteria of Escherichia coli carrying antigens of the other type.

15. A process according to any one of claims 10 to 13, characterised in that one uses as antigens of the other type a suspension of antigen substantially free from bacteria.